# EUROPÄISCHE PATENTSCHRIFT

(11) **EP 3 540 431 B1**
(45) Veröffentlichungstag und Bekanntmachung des Hinweises auf die Patenterteilung: **05.05.2021**
(21) Anmeldenummer: 19162102.8
(22) Anmeldetag: 12.03.2019
(51) Int. Cl.: G01N 33/18, G01N 31/12

(54) **MESSANORDNUNG UND MESSVERFAHREN ZUR BESTIMMUNG EINES INHALTSSTOFFES ODER QUALITÄTSPARAMETERS VON WASSER ODER ABWASSER**
MEASURING ASSEMBLY AND MEASURING METHOD FOR DETERMINING A COMPONENT OR QUALITY PARAMETER OF WATER OR WASTE WATER
DISPOSITIF DE MESURE ET PROCÉDÉ DE MESURE PERMETTANT DE DÉTERMINER UN INGRÉDIENT OU UN PARAMÈTRE DE QUALITÉ DE L'EAU OU DES EAUX USÉES

(30) Priorität: 12.03.2018 DE 102018105611
(43) Veröffentlichungstag der Anmeldung: 18.09.2019
(73) Patentinhaber: LAR Process Analysers AG, 12057 Berlin (DE)
(72) Erfinder: Arts, Werner, 10825 Berlin (DE); Genthe, Wolfgang, 14059 Berlin (DE)
(74) Vertreter: Heinze, Ekkehard

(56) Entgegenhaltungen:
- EP-A1- 1 055 927
- EP-A2- 0 647 848
- EP-B1- 2 115 453

## Beschreibung

Die Erfindung betrifft eine Messanordnung und/oder ein Messverfahren zur Bestimmung eines Inhaltsstoffes oder Qualitätsparameters von Wasser oder Abwasser.

Es ist bekannt, zur Bestimmung des Gehaltes an bestimmten Wasserinhaltsstoffen - und damit der Qualität von Trinkwasser, Prozesswasser oder auch Meerwasser und von durch organische Stoffe, Stickstoffverbindungen o.ä. belastetem Abwasser - eine Probe in einer Atmosphäre eines mit Sauerstoff angereicherten inerten Transportgases (Trägergases) zu verdampfen und zu verbrennen und das hierbei erhaltene Verbrennungsgasgemisch einem zum Nachweis von Kohlendioxid, Stickoxiden etc. geeigneten Detektor zuzuführen.

Als Detektoren haben sich (neben anderen) Infrarotdetektoren für den Kohlenstoffgehalt, spezielle Chemolumineszenzdetektoren respektive elektrochemische Sensoren für den Stickstoffgehalt und sogenannte coulometrische Detektoren für den Halogenidgehalt bewährt.

Große Verbreitung haben die auf der Verbrennung einer Wasserprobe beruhenden Nachweisverfahren für die Erfassung des Gehaltes an organischen Inhaltsstoffen - des sogenannten TOC (total organic carbon) - erlangt. Hierbei wird üblicherweise eine kleine Wassermenge mit dem Transportgas einem mit einer Widerstandsheizung auf eine vorbestimmte Temperatur aufgeheizten Ofen zugeführt, wo sie nahezu schlagartig verdampft und verbrennt, und das Verbrennungsgas wird einem NDIR-CO₂-Detektor zugeführt, dessen CO₂-Gehaltsanzeige ein Maß für den C-Gehalt des Wasserprobe bildet. Eine fortgeschrittene Ausführung dieses Verfahrens und eine entsprechende Apparatur sind in DE 43 44 441 C2 beschrieben. Eine zur Messung sehr niedriger TOC-Werte - etwa in hochreinem Wasser bzw. hochreinen Lösungen für medizinische Anwendungen - modifizierte Anordnung ist in EP 0 684 471 A2 beschrieben.

Weiterentwickelte Verfahren dieser Art und zweckmäßig ausgestaltete Reaktoren bzw. Gesamtanordnungen hat die Anmelderin in der EP 0 887 643 B1 und der EP 1 055 927 B1 vorgeschlagen. Eine verbesserte Probenbeschickung bei einer solchen Messanordnung ist Gegenstand der WO 2016/091252 A2 der Anmelderin.

Die US 5702954 beschreibt ein mehrstufiges Aufschlussverfahren für pflanzliche oder tierische phosphorhaltige Proben des Phosphatgehaltes, welches eine Verbrennung in Gegenwart eins Reduktionsmittels (etwa Wasserstoff) und eine anschließende Umsetzung mit Ozon in einer weiteren Reaktionskammer bei Umgebungstemperatur einschließt. Auch die US 2003/0032194 A1 beschreibt ein mehrstufiges Oxidationsverfahren, welches primär zur Bestimmung von Stickstoff und Schwefel, aber auch von Phosphor, in einer diese Elemente enthaltenden Probe entwickelt wurde. Thermische Aufschlussverfahren unter Einsatz spezieller Katalysatoren bzw. von Ozon sind etwa auch aus der JP 59154358 A oder JP 61140863 A bekannt.

Eine Messanordnung und ein Messverfahren zur Bestimmung des Phosphorgehaltes von Abwasserproben, das auf den oben genannten Patenten/Anmeldungen der Anmelderin basiert, ist in der EP 2 115 453 B1 der Anmelderin beschrieben.

Der Erfindung liegt die Aufgabe zu Grunde, eine verbesserte Messanordnung und ein verbessertes Messverfahren anzugeben, die für verschiedene Wasserinhaltsstoffe bzw. Qualitätsparameter einsetzbar sind, einen kostengünstigen Aufschluss der Proben erlauben und in der Praxis leicht und sicher handhabbar sind.

Diese Aufgabe wird in ihrem Vorrichtungsaspekt durch eine Anordnung mit den Merkmalen des Anspruchs 1 und in ihrem Verfahrensaspekt durch ein Verfahren mit den Merkmalen des Anspruchs 6 gelöst. Zweckmäßige Fortbildungen des Erfindungsgedankens sind Gegenstand der abhängigen Ansprüche.

Die Erfindung schließt den Gedanken ein, die Wärmeverluste am Reaktionsmodul der Messanordnung durch geeignete konstruktive und Steuerungs-Maßnahmen zu verringern und insbesondere Gehäusetemperaturen des Reaktionsmodules in dessen bei Bedienvorgängen kritischen Kopf- und Fußbereich zu begrenzen. Gleichzeitig soll die für einen effizienten Probenaufschluss erforderliche Maximaltemperatur in der Reaktionszone gewährleistet sein. Speziell zeichnet sich das vorgeschlagene Reaktionsmodul und dessen Betriebsverfahren dadurch aus, dass das Reaktionsmodul, die Widerstands- oder Infrarotheizung und Mittel zur Proben- und Trägergaszuführung derart ausgebildet sind, dass im Betrieb der Messanordnung eine Außen-Kopftemperatur T_{H} ≤ 80°C und eine Außen-Fußtemperatur T_{F} ≤ 150°C bei einer Maximaltemperatur in der Reaktionszone T_{MAX} ≥ 1150°C beträgt.

Mit diesen Maßnahmen wird zum einen eine erhebliche Reduzierung des Energieverbrauchs des Reaktionsmodules und damit insgesamt der Messanordnung erreicht, was insbesondere bei einem mobilen Betrieb mit Akkus oder Batterien einen erheblichen Gebrauchswertvorteil für die Nutzer darstellt. Zum anderen wird die Handhabung der Messanordnung dadurch noch sicherer und einfacher, und auch dies stellt einen erheblichen Nutzer-Vorteil dar.

In einer Ausführung der Erfindung sind das Reaktionsmodul, die Widerstands- oder Infrarotheizung und Mittel zur Proben- und Trägergaszuführung derart ausgebildet, dass im Betrieb der Messanordnung eine Kopftemperatur T_{H} ≤ 70°C und eine Fußtemperatur T_{F} ≤ 150°C, insbesondere ≤ 120°C, beträgt, wobei insbesondere die Maximaltemperatur in der Reaktionszone T_{MAX} ≥ 1200°C beträgt.

Das vorgeschlagene Reaktionsmodul und Betriebsverfahren sind in modularer Weise in verschiedenen Messanordnungen einsetzbar, welche unter anderem zur Bestimmung von Stickstoff und/oder Phosphor und/oder des Gehaltes an organischem Kohlenstoff, TOC, oder des chemischen Sauerstoffbedarfs, CSB, ausgestaltet sind.

In einer weiteren zweckmäßigen Ausführung ist das Reaktionsmodul zum überwiegenden Teil mit einer Füllung aus porösen Keramikkugeln befüllt. Durch die Auswahl geeigneter Keramikkugeln, mit auf den Anwendungsfall abgestimmter Größe und Porosität, lässt sich der Durchfluss bzw. die Verweildauer der Probe in der Reaktionszone und ggfs. speziell auch die Verweildauer in verschiedenen Temperaturbereichen der Reaktionszone und damit insgesamt das T-Regime des Reaktionsmoduls optimieren. In praxisgerechten Ausführungen werden Al₂O₃-Kugeln eingesetzt, die speziell mittlere Durchmesser von 6, 4,5, 2,7 und 1,2mm haben, wobei mindestens zwei, bevorzugt vier Schichten von Kugeln mit jeweils unterschiedlichem Durchmesser übereinander liegen und die Dicke der einzelnen Schichten im Hinblick auf die konkreten Anwendungs- und Verfahrensbedingungen derart gewählt werden, dass die Oxidation der Wasserinhaltsstoffe zu den Analyten im Messgas in den verschiedenen Temperaturzonen des Reaktors sicher gewährleistet ist.

In der Praxis haben sich Ausführungen als zweckmäßig erwiesen, bei denen das Reaktionsmodul eine sich mindestens über die Länge der Reaktionszone erstreckende Innenwandung (bzw. ein separates Reaktionsgefäß) aus Al2O3 und im Kopf- und Fußbereich jeweils einen FCKW-Einsatz mit einem eine Teflon-Ummantelung aufweisenden O-Ring als Dichtelement aufweist.

Aluminiumoxid als Reaktormaterial hat eine für die Betriebsbedingungen ausreichende Temperaturbeständigkeit und Wärmeleitfähigkeit aufweist und darüber hinaus eine für einen praktischen Betrieb ausreichende Temperaturwechselbeständigkeit. Bei einer möglichen Verringerung der Aufschlusstemperatur kann auch ein Hochleistungsedelstahl verwendet werden. Der Ofenkopf besteht insbesondere aus einem mechanisch verstärkten Fluorkohlenwasserstoff zur Wärmeisolation. Der Ofenfuß besteht applikationsbedingt entweder aus einem mechanisch verstärktem Fluorkohlenwasserstoff, oder aus einem Glasfuß mit Keramik-Prallplatte zur Wärmeumleitung des heißen Reaktionsgases.

In praxisgerechten Ausführungen weist der Kopfabschnitt des Reaktionsmodules einen Injektionsport zum temporären Einführen einer Injektionsnadel oder zur dauerhaften Halterung eines Zuleitungsröhrchens auf, der insbesondere einen durch eine Silikoneinlage oder eingelegte Feder innengefederten O-Ring umfasst.

In Anpassung an diese Ausführung umfasst die Messanordnung speziell eine durch eine Druckfeder oder einen Schrittmotor betriebene Injektionsspritze zum Eintrag der Probe in das Reaktionsmodul. Diese Art der Beschickung des Reaktionsmodules mit einer Probe ist als solche, insbesondere aus früheren Schutzrechten/Anmeldungen der Anmelderin, bekannt. Für Details dieser Beschickungsmehtode kann daher auf den Stand der Technik, etwa die WO 2016/091252 A2, verwiesen werden. Hierbei ist insbesondere der Druckfeder oder dem Schrittmotor ein Detektor zur Erfassung des Beginns eines Einspritzvorganges der Probe in das Reaktionsmodul zugeordnet. Dieser Gedanke einer Detektion des Einspritzvorganges, in Verbindung mit der weiter unten beschriebenen Verwendung des Detektionssignals ist neu.

Eine alternative Ausführung des Reaktionsmodules weist ein mit dem Zuleitungsröhrchen verbundenes Dreiwegeventil zur wahlweisen Einleitung von Probe oder einer Spülflüssigkeit in das Reaktionsmodul auf. Auch hier ist dem die Probenzuführung bewirkenden Element, also dem Dreiwegeventil, bevorzugt ein Detektor zur Erfassung des Beginns eines Einspritzvorganges der Probe in das Reaktionsmodul zugeordnet. Diese Ausführung hat nach Untersuchungen der Erfinder Vorteile hinsichtlich der Verschleißbeständigkeit der Dichtung(en) und damit des Wartungsaufwandes für die Messanordnung, kann aber auch hinsichtlich der Schnelligkeit der Probenzuführung und der raschen alternierenden Ausführung von Mess- und Spülvorgängen vorteilhaft sein.

In weiteren Ausführungen sind am Injektionsport Einstellmittel zur Einstellung der Position des Endes der Injektionsnadel oder des Zuleitungsröhrchens im Reaktionsmodul vorgesehen. Durch derartige Einstellungen lassen sich in gewissem Umfang der Temperaturverlauf im Reaktionsmodul und insbesondere auch die Temperaturen an dessen Kopf- und Fußbereich beeinflussen.

Ebenfalls dem Ziel einer gezielten Einstellung des Temperaturverlaufes dient eine Ausführung, bei der die Widerstands- oder Infrarotheizung eine Mehrzahl von vertikal gereihten, separat angesteuerten Heizelementen, insbesondere separaten Widerstandsheiz-/Keramikisoliermodulen, umfasst. Insbesondere ist hierbei mindestens einem der Heizelemente ein Temperatursensor und ein entsprechender Steuereingang einer Heizsteuereinrichtung zugeordnet und die Heizsteuereinrichtung ist derart ausgebildet, dass die Heizelemente, insbesondere einzeln, in Abhängigkeit von einem Ausgangssignal des Temperatursensors und gemäß einem vorbestimmten Temperaturprofil des Reaktionsmodules mit Heizstrom beaufschlagt werden können.

In einer weiteren Ausführung hat eine Heizsteuereinrichtung der Widerstands- oder Infrarotheizung einen Detektoreingang zum Empfang eines einen laufenden Eintragvorganges einer Probe repräsentierenden Einspritz-Eingangssignals auf, und die Heizsteuereinrichtung ist derart ausgebildet, dass sie aufgrund des Einspritz-Eingangssignals die Heizleistung der Widerstands- oder Infrarotheizung variiert. Diese Maßnahme kann insbesondere vorteilhaft mit der vorgenannten Maßnahme einer Temperatursensor-Steuerung der Widerstands- bzw. Infrarotheizung kombiniert sein, um somit dem Einfluss des Probeneinspritzvorganges auf die Temperaturverteilung im Reaktionsmodul differenziert Rechnung zu tragen.

Nach den Untersuchungen der Erfinder ist vorteilhafterweise in der Heizsteuereinrichtung ein PID-Regelalgorithmus implementiert, der zumindest das Ausgangssignal eines Temperatursensors und optional das Einspritz-Eingangssignal zur Temperaturregelung im Reaktionsmodul nutzt.

Die Steuerung der Messanordnung kann andererseits dahingehend ausgestaltet sein, dass die Mittel zur Proben- und Trägergaszuführung eine Zuführ-Steuereinrichtung zur automatisch gesteuerten Proben- und Trägergaszuführung aufweisen, die insbesondere mindestens einen Eingangsanschluss zum Empfang eines von der Heizsteuereinrichtung gelieferten Eingangssignal zur Beeinflussung der automatisierten Proben- und Trägergaszuführung aufweist. Hierbei wird also gewissermaßen die Probeneinspritzung unter Beachtung der aktuellen Temperaturverhältnisse im Reaktionsmodul gesteuert und ggfs. gegenüber einem Standard-Regime variiert.

Verfahrensaspekte der Erfindung ergeben sich weitgehend aus den oben erläuterten Vorrichtungsaspekten und werden insoweit hier nicht nochmals genauer beschrieben.

Hingewiesen wird jedoch auf den Aspekt, dass beim Eintragvorgang einer Probe in das Reaktionsmodul ein Einspritz-Eingangssignal für eine Heizsteuereinrichtung der Widerstands- oder Infrarotheizung erzeugt und die Heizsteuereinrichtung derart betrieben wird, dass sie aufgrund des Einspritz-Eingangssignals die Heizleistung der Widerstands- oder Infrarotheizung variiert.

Weiterhin wird auf den Aspekt hingewiesen, dass Heizelementen der Widerstands- oder Infrarotheizung mindestens ein Temperatursensor zugeordnet ist und die Signale des oder jedes Temperatursensors entsprechenden Steuereingängen der Heizsteuereinrichtung zugeführt werden und die Heizsteuereinrichtung derart betrieben wird, dass die Heizelemente in Abhängigkeit von einem Ausgangssignal des zugehörigen Temperatursensors und gemäß einem vorbestimmten Temperaturprofil des Reaktionsmodules mit Heizstrom beaufschlagt werden.

Hingewiesen wird weiterhin darauf, dass vorteilhaft in der Heizsteuereinrichtung ein PID-Regelalgorithmus derart betrieben wird, dass dieser zumindest das Ausgangssignal eines Temperatursensors und optional das Einspritz-Eingangssignal zur Temperaturregelung im Reaktionsmodul nutzt. Schließlich kann vorgesehen sein, dass die Mittel zur Proben- und Trägergaszuführung eine Zuführ-Steuereinrichtung zur automatisch gesteuerten Proben- und Trägergaszuführung aufweisen und derart betrieben werden, dass Schwankungen der Temperatur in der Reaktionszone minimiert werden.

Vorteile und Zweckmäßigkeiten der Erfindung ergeben sich im Übrigen aus der nachfolgenden Beschreibung eines Ausführungsbeispiels und wesentlicher Ausführungsaspekte der Erfindung anhand der Figuren. Von diesen zeigen:
- Fig. 1: eine skizzenartige Gesamtdarstellung einer erfindungsgemäßen Anordnung,
- Fig. 2: eine schematische Querschnittsdarstellung der wesentlichen Abschnitte eines Reaktionsmoduls einer Messanordnung gemäß einer Ausführungsform der Erfindung,
- Fig. 3: eine schematische Längsschnittdarstellung einer Injektionsspritze mit in den Injektionsport eingeführter Injektionsnadel gemäß einer beispielhaften Ausführungsform der Analyseanordnung und
- Fig. 4: eine Prinzipskizze einer weiteren Ausführung der Probenbeschickung einer erfindungsgemäßen Messanordnung.

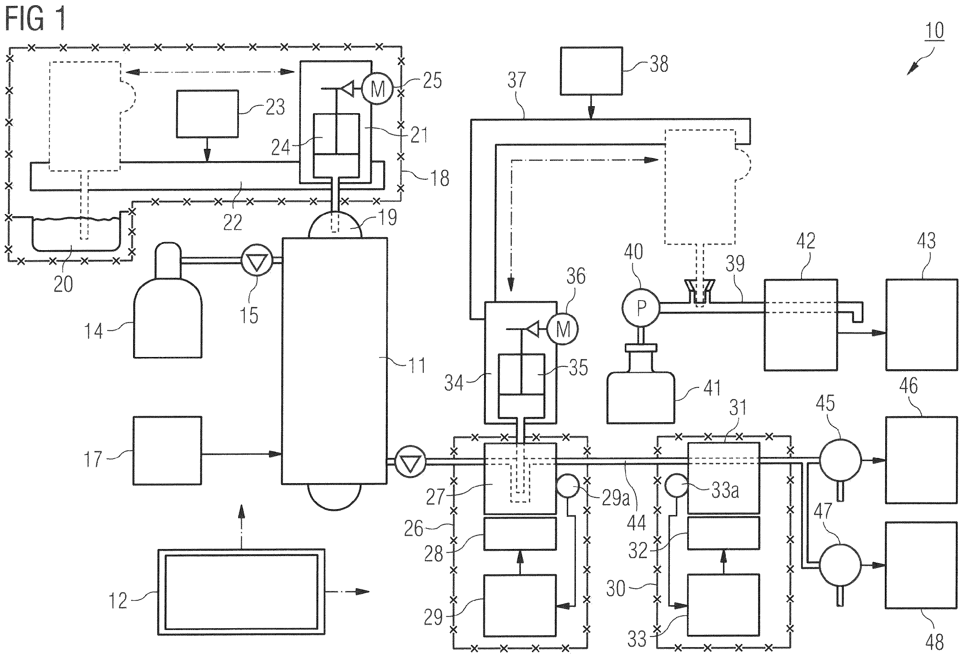

Fig. 1 zeigt in Art einer Prinzipskizze den Gesamtaufbau einer beispielhaften Messanordnung 10 zur Bestimmung verschiedener Wasserinhaltsstoffe von Abwasser bzw. Brauchwasser. Hauptkomponente der Anordnung 10 ist ein weiter unten beschriebenes Reaktionsmodul 11; es kann aber auch ein anderer Typ von Verbrennungsofen (etwa mit Strahlungsheizung) an dessen Stelle eingesetzt sein. Im Interesse einer besseren Übersichtlichkeit der Darstellung sind nicht erfindungswesentliche Teile, die etwa der Kalibrierung und Reinigung der Messanordnung dienen, in dieser skizzenhaften Darstellung fortgelassen.

Eine symbolisch dargestellte Steuereinheit (Controller) 12 steuert den Gesamtablauf des Probenaufschlusses und der Messvorgänge und ist zu diesem Zwecke selbstverständlich mit den wesentlichen Absperr-, Transport-, Heiz- und Nachweiseinrichtungen der Anordnung verbunden. Implementierung, Anschluss und Betrieb einer solchen Steuereinrichtung liegen, auf der Grundlage der weiter unten gegebenen Verfahrensbeschreibung und des nachfolgend erläuternden Vorrichtungsaufbaus, im Rahmen fachmännischen Handelns.

Dem Reaktionsmodul 11 ist eingangsseitig ein Trägergasspeicher 14 zur Bereitstellung von Trägergas für die Messvorgänge, mit zugeordneter Eingangs-Ventileinrichtung 15 zugeordnet. Weiterhin hat der Ofen eine Heizsteuereinheit 17 zur Steuerung der elektrischen Ofenheizung und eine Probenzuführeinrichtung 18 zur Probenzuführung in ein Probeneinspritzventil 19 des Ofens.

Die Probenzuführeinrichtung 18 umfasst ein Probenreservoir 20, welches etwa am Einlauf einer Kläranlage angeordnet sein kann, eine Injektionseinheit 21, die auf einer Transportführung 22 verfahrbar gelagert ist und eine zugehörige Transportsteuerung 23. Die Spritzeneinheit 21 umfasst eine Dosierspritze 24 und einen Schrittmotor 25 zu deren präzise steuerbarer Betätigung und damit Dosierung eines vorbestimmten Probenvolumens.

Am Ausgang des Reaktionsmodules 11 ist eine erste Kühlstufe 26 angeordnet, die eine Kühlfalleneinheit 27, einen Peltierkühler 28 und eine zugehörige Temperatursteuerung 29 mit T-Sensor 29a an oder in der Kühlfalleneinheit 27 umfasst. Stromabwärts der ersten Kühlstufe 26 ist eine zweite Kühlstufe 30 angeordnet, welche einen Kühlblock 31 mit zugeordnetem Peltierkühler 32 und eine diesen steuernde Temperatursteuereinheit 33 mit T-Sensor 33a umfasst.

Der ersten Kühlstufe 26 ist eine weitere Spritzeneinheit 34 zugeordnet, die - analog zur Spritzeneinheit 21 zur Beschickung des Verbrennungsofens 1 - eine Injektionsspritze 35 mit Schrittmotor 36 zu deren präzise gesteuerter Betätigung aufweist. Des Weiteren ist auch diese Spritzeneinheit 34 auf einer Transportführung 37 gelagert, der eine Transportsteuereinheit 38 zum Verfahren der Spritzeneinheit in eine zweite Betriebsposition zugeordnet ist. Diese befindet sich oberhalb einer Durchflussküvette 39, in die die Nadel der Injektionsspritze 35 ebenso eingreifen kann wie in die Kühlfalle 27. Diese zweite Betriebsstellung ist in der Figur, ebenso wie die Ausgangs-Betriebsstellung der Spritzeneinheit 21, gestrichelt dargestellt.

An einem Eingang der Durchflussküvette 39 ist über eine Pumpe 40 ein Reagensbehälter 41 angeschlossen, in dem eine für den photometrischen Phosphor-Nachweis benötigte Chemikalie bevorratet ist. Die Durchflussküvette 39 ragt in eine Photometereinheit 42, die zur photometrischen Analyse einer die Durchflussküvette 39 durchströmenden wässrigen Probe ausgebildet ist und deren Ausgang mit einer Phosphor-Auswertungsstufe 43 verbunden ist.

Am Ausgang der zweiten Kühlstufe 30 verzweigt sich die Ausgangsleitung 44 des Verbrennungsofens 1 zu einem NO-Detektor 45, der ausgangsseitig mit einer Stickstoff(TN)-Auswertungsstufe 46 verbunden ist, und zu einem CO₂-Detektor 47, welcher ausgangsseitig mit einer Kohlenstoff(TOC)-Auswertungsstufe 48 verbunden ist.

Die Funktionsweise der Messanordnung 13 ergibt sich teilweise aus den obigen Erläuterungen zum erfindungsgemäßen Verfahren, soll aber nachfolgend nochmals knapp zusammengefasst werden.

Mittels der ersten Spritzeneinheit 21 wird eine wässrige Probe aus dem Reservoir 20 entnommen, zum Verbrennungsofen 1 transportiert und in diesen eingespritzt. Sie wird bei den dort eingestellten Temperaturen augenblicklich verdampft und verbrannt, und das entstehende Verbrennungsgas wird mit einem aus dem Trägergas-Reservoir 14 gespeisten Trägergasstrom aus dem Ofen in die Ausgangsleitung 44 geführt. Im Kondensator wird der Verbrennungsgas-/Trägergasstrom auf eine erste Abkühltemperatur heruntergekühlt, bei der sich in der Kühlfalle 27 ein Kondensat abscheidet. Von diesem Kondensat wird mittels der zweiten Spritzeneinheit 34 eine vorbestimmte Menge abgezogen und in die Durchflussküvette 39 gebracht, wo es mit dem über die Pumpe 40 geförderten Reagens zum Bewirken eines photometrischen Nachweisvorganges vermischt und der Photometereinheit 42 zur Phosphor-Detektion zugeführt wird.

In der zweiten Kühlstufe 30 wird der Verbrennungsgas-/Trägergasstrom auf eine nahe 0°C liegende zweite Abkühltemperatur heruntergekühlt und ausgangsseitig der Kühlstufe den Gasdetektoren 45 und 46 für den NO- und CO₂-Nachweis zugeführt. Im Ergebnis der Nachweisvorgänge an den Detektoren 42, 45 und 47 ermitteln die entsprechenden Auswertungsstufen 43, 46 und 48 den Gesamt-Phosphorgehalt (TP), den Gesamt-Stickstoffgehalt (TN) und den Gesamtgehalt an organischem Kohlenstoff (TOC) der wässrigen Probe, die aus dem Reservoir 20 entnommen und im Verbrennungsofen 1 aufgeschlossen wurde.

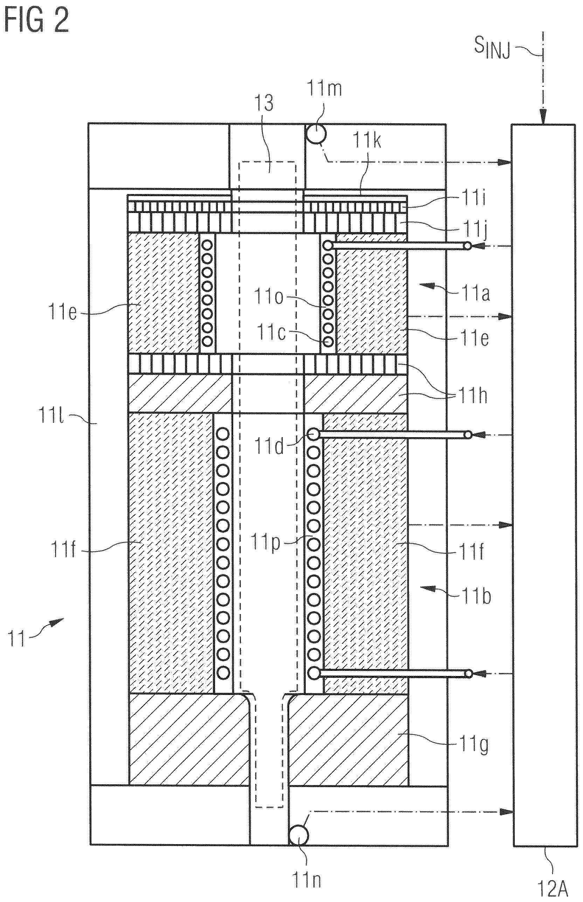

Fig. 2 zeigt in einer schematischen Querschnittsdarstellung wesentliche Abschnitte eines Probenverbrennungsofens (Reaktionsmoduls) 11 in einer erfindungsgemäßen Ausführung, in den ein im wesentlichen langgestreckt zylindrisches Edelstahl-Reaktionsgefäß 13 (in der Figur mit einer gestrichelten Linie umrissartig gezeichnet) einsetzbar ist. Dieses hat am unteren Ende (Fußende) einen rohrförmigen Auslass, der zur Beseitigung von Salz-Ablagerungen leicht von unten her gereinigt werden kann.

Der Ofen 11 weist in der gezeigten speziellen Zwei-Zonen-Konfiguration eine erste, obere Heizzone 11a, in der bei dieser Ausführung eine Maximaltemperatur von 800°C erreicht werden kann, und eine zweite, untere Heizzone 11b auf, in der eine Maximaltemperatur von 1250°C erreicht wird. Beide Heizzonen werden über hohlzylindrisch um den entsprechenden Abschnitt des Reaktionsgefäßes 13 angeordnete Heizmodule 11c, 11d aus einer hochtemperaturfesten Speziallegierung, etwa dem Material Kanthal-Fibrothal®, beheizt.

Die Heizmodule 11c, 11d weisen - wegen der unterschiedlichen Maximaltemperaturen unterschiedlich dicke - mikroporöse Keramikfaser-Isolationen 11e bzw. 11f auf, und auch der Fußbereich 119, der Bereich 11h zwischen den Heizzonen und der Kopf-Bereich 11i, 11j unterhalb eines Aluminiumdeckels 11k sind keramikfaserisoliert. Eine (in der Figur nicht dargestellte) Probenbeschickungs- und Trägergaszuführungseinrichtung ist im Bereich oberhalb des Deckels 11k vorgesehen. Das gesamte Reaktionsmodul 11 ist zusätzlich mit einer makroporösen Außenisolierung 11l ummantelt, die sowohl die Temperatur am Außenumfang des Ofens als auch an dessen Kopf und Fuß deutlich senkt, und zwar im Normalbetrieb des Ofens auf die weiter oben genannten Werte.

Der Verbrennungsofen 11 weist eine komplexe Temperatursensorik auf, die ebenfalls einen Beitrag zur Erreichung dieses Ziels leisten kann. Diese umfasst jeweils einen im Kopf- und Fußbereich angeordneten T-Sensor 11m, 11n sowie jeweils einen den Heizmodulen 11c, 11d zugeordneten T-Sensor 11o, 11p. Alle T-Sensoren sind mit entsprechenden Eingängen einer Heizsteuer- und Regeleinrichtung verbunden, die in Anlehnung an die Bezeichnung des Controllers 12 in Fig. 1 mit 12A bezeichnet ist. In der Heizsteuer- und Regeleinrichtung 12A werden die Detektionssignale der T-Sensoren gemäß einem gespeicherten Optimierungsalgorithmus (ggfs. zusammen mit Signalen S_{INJ}, die einen Probeneinspritzvorgang kennzeichnen (siehe weiter unten) zu Ansteuersignalen für die Heizmodule 11c, 11d verarbeitet, die die Stromzuführung zu diesen und damit zeitabhängig die Beheizung der Heizzonen 11a, 11b steuern. Vorteilhaft ist in der Heizsteuer- und Regeleinrichtung 12a ein PID-Regelalgorithmus implementiert.

Der in Fig. 2 gezeigte und vorstehend beschriebene Ofenaufbau trägt zusammen mit der beschriebenen selektiven und geregelten Beheizung in vorteilhafter Weise zur dauerhaften Realisierung speziell der in der zweiten, unteren Heizzone 11c erzeugten hohen Temperaturen von über 1200°C bei, wobei die spezielle Isolierung sowohl zu einem vertretbaren Energieaufwand beiträgt als auch Gefährdungen der Umgebung ausschließt.

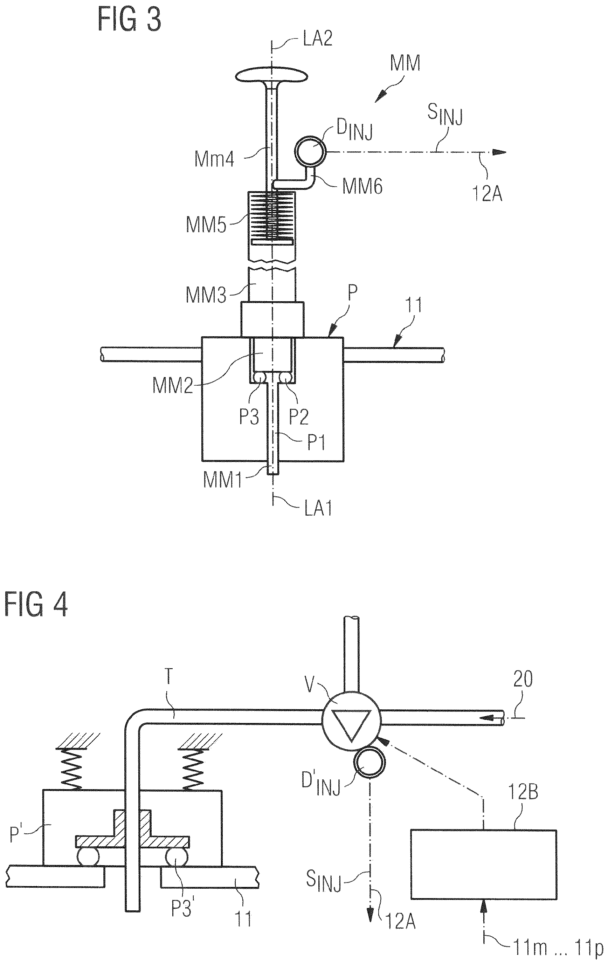

Fig. 3 zeigt skizzenartig in einer Längsschnittdarstellung einen beispielhaften Aufbau und die aufeinander abgestimmte geometrische Konfiguration der Injektionsspritze MM und des Injektionsports P des Ofens 11 (Fig. 2) der Messanordnung 10 (Fig. 1).

Der Injektionsport P umfasst eine in ihrem Längsverlauf im Wesentlichen zylindrisch ausgebildete Führungshülse P1, deren Durchmesser und Länge auf die entsprechenden Abmessungen einer Injektionsnadel MM1 der Injektionsspritze MM abgestimmt sind und deren Längsachse mit einer Längsachse LA1 des in seiner Grundform zylindrisch ausgeführten Ofens zusammenfällt. An der Oberseite des Injektionsports P ist eine Bohrung P2 mit vergrößertem Durchmesser vorgesehen, deren Abmessungen auf diejenigen eines Nadel-Ansatzes MM2 der Injektionsspritze angepasst sind und deren untere Stirnfläche beim Einführen der Injektionsspritze als Anschlag zur Tiefenbegrenzung wirkt. Auf der unteren Stirnfläche der Bohrung P2 liegt als Dichtung ein O-Ring P3, der insbesondere als Silikonring mit Teflonbeschichtung ausgeführt sein kann. Mit diesem Anschlag wird eine exakt vorbestimmte Position des rechtwinklig zur Nadel-Längsachse LA2 der Injektionsspritze abgeschnittenen Nadelendes im Ofen 11 und somit ein exakt vorbestimmter Einspritzpunkt gewährleistet.

Im Spritzen-Reservoir MM3 ist ein Spritzenkolben MM4 längsverschieblich gelagert, dessen freies Ende in üblicher Weise zum manuellen Aufziehen einer Probe ausgestaltet ist. Am oberen Ende des Spritzenreservoirs ist in diesem eine Druckfeder MM5 eingebettet, deren oberes Ende sich gegen die obere Stirnwand des Spritzenreservoirs abstützt und deren unteres Ende auf das Ende des Spritzenkolbens MM4 wirkt. Nach dem Aufziehen der Spritze wird mittels eines Verriegelungshebels MM6 der Spritzenkolben mit gespannter Feder MM5 arretiert. Nach Lösen der Verriegelung MM6 wird der Spritzenkolben MM4 durch die Kraft der Druckfeder MM5 nach unten gedrückt und die im Spritzenreservoir MM3 enthaltene Probe in einem vorbestimmten Zeitintervall bzw. mit vorbestimmter Austraggeschwindigkeit in den Ofen eingespritzt.

Dieser Austrag der vorbestimmten Probenmenge mit exakt vorbestimmter Geschwindigkeit bzw. in einem exakt definierten Zeitintervall ist für reproduzierbare Analyseergebnisse ebenso wichtig wie die exakte Einspritzposition- und Richtung, die durch die spezielle Gestaltung der Injektionsnadel und des Injektionsports gesichert werden. In einer nicht dargestellten Ausführung kann ein verstellbarer Anschlag oder auch eine andersartige Einrichtung zur Verstellung der Position des Endes der Injektionsnadel im Ofen vorgesehen sein, was auch im Kontext einer optimierten Temperatursteuerung eine Rolle spielen kann.

In diesem Kontext steht auch das Vorsehen eines Positionsdetektors D_{INJ} am Verriegelungshebel MM6 der Injektionsspritze MM, der die Stellung des Verriegelungshebels und somit die erfolgte Freigabe der Druckfeder MM5 und somit wiederum einen eingeleiteten Einspritzvorgang detektiert. Der Detektor D_{INJ} liefert ein Einspritz-Eingangssignal S_{INJ} an die Heizsteuer- und Regeleinrichtung 12A (Fig. 2), die dieses Signal für die Bereitstellung eines Ansteuersignals (Zeitpunkt und Stromstärke) für die Heizmodule des Ofens verarbeiten kann. Die zeitgesteuerte bzw. geregelte Beheizung des Ofens berücksichtigt somit Einspritzvorgänge, die zu "Temperaturstößen" im Ofen führen können, im Sinne einer Glättung des zeitlichen Temperaturverlaufs und der Vermeidung von Temperaturspitzen oberhalb der gewünschten Maximalwerte am Ofenfuß.

In ähnlicher Weise funktioniert eine in Fig. 4 skizzenartig dargestellte alternative Ausführungsform der Probenbeschickung des Reaktionsmoduls (Verbrennungsofens). Bei dieser wird anstelle einer verfahrbaren Injektionsspritze ein dauerhaft am Injektionsport P' installiertes Zuleitungsröhrchen T in Verbindung mit einem Absperr- oder Dreiwegeventil V eingesetzt. Hier kann die Ventilposition und somit ein stattfindender Einspritzvorgang mittels eines ähnlichen Positionsdetektors D'_{INJ} wie bei Fig. 3 erfasst werden, oder es ist am Zuleitungsröhrchen T ein Durchflussdetektor vorgesehen, der ebenfalls einen Einspritzvorgang erfasst und ein entsprechendes Einspritz-Eingangssignal für die Heizsteuer- und Regeleinrichtung der Messanordnung liefert.

Im Übrigen zeigt Fig. 4 schematisch, dass die Messanordnung eine Zuführ-Steuereinrichtung zur Steuerung der Probeneinspritzung umfassen kann, die in Anlehnung an die Bezeichnung des Controllers 12 in Fig. 1 mit Ziffer 12B bezeichnet ist. Dieser können die Temperatursignale einzelner oder aller T-Sensoren 11m bis 11p (Fig. 2) zugeführt werden, um in Abhängigkeit von den T-Signalen , also vom aktuellen Temperatur-Status des Verbrennungsofens 11, Einspritzvorgänge durch Betätigung des Ventils V differenziert zu steuern.

## Patentansprüche

1. Messanordnung zur Bestimmung eines Inhaltsstoffes und/oder eines Qualitätsparameters von Wasser oder Abwasser, umfassend ein Reaktionsmodul (11) zum thermischen Aufschluss einer Probe des Wassers oder Abwassers, Mittel zur Zuführung des Reaktionsproduktes zu einem Detektor in einem Trägergasstrom, den Detektor (42, 45, 47) und eine Auswertungseinrichtung (43, 46, 48) zur Auswertung eines Detektorsignals zur Ableitung eines Wertes des Inhaltsstoffes oder Qualitätsparameters,
wobei das Reaktionsmodul ein langgestrecktes, im Betrieb vertikal ausgerichtetes Gefäß mit Widerstands- oder Infrarotheizung ist und einen Kopfabschnitt, in dem die Probe eingetragen wird, eine Reaktionszone, in der der thermische Aufschluss erfolgt, sowie einen Fußabschnitt hat, aus dem das Reaktionsprodukt im Trägergasstrom ausgetragen wird,
wobei das Reaktionsmodul, die Widerstands- oder Infrarotheizung und Mittel zur Proben- und Trägergaszuführung derart ausgebildet sind, dass im Betrieb der Messanordnung eine Außen-Kopftemperatur T_{H} ≤ 80°C und eine Außen-Fußtemperatur T_{F} ≤ 150°C bei einer Maximaltemperatur in der Reaktionszone T_{MAX} ≥ 1150°C beträgt, wobei
das Reaktionsmodul in einer Zwei-Zonen-Konfiguration eine erste, obere Heizzone (11a), die zur Erreichung einer Maximaltemperatur von 800°C konfiguriert ist, und eine zweite, untere Heizzone (11b) aufweist, die zur Erreichung einer Maximaltemperatur von 1250°C konfiguriert ist,
**dadurch gekennzeichnet, dass**
die erste und zweite Heizzone über hohlzylindrisch um den entsprechenden Abschnitt des Reaktionsmoduls angeordnete Heizmodule (11c, 11d) aus einer hochtemperaturfesten Speziallegierung beheizt sind, und dass die Heizmodule (11c, 11d) unterschiedlich dicke mikroporöse Keramikfaser-Isolationen (11e, 11f) aufweisen und auch der Fußabschnitt (11g), ein Bereich (11h) zwischen den Heizzonen und der Kopfabschnitt, (11i, 11j) unterhalb eines Aluminiumdeckels (11k) keramikfaserisoliert sind und das gesamte Reaktionsmodul (11) zusätzlich mit einer makroporösen Außenisolierung (11l) ummantelt ist.

2. Messanordnung nach Anspruch 1, ausgebildet zur Bestimmung von Stickstoff und/oder Phosphor und/oder des Gehaltes an organischem Kohlenstoff, TOC, oder des chemischen Sauerstoffbedarfs, CSB.

3. Messanordnung nach Anspruch 1 oder 2, wobei das Reaktionsmodul, die Widerstands- oder Infrarotheizung und Mittel zur Proben- und Trägergaszuführung derart ausgebildet sind, dass im Betrieb der Messanordnung eine Kopftemperatur TH ≤ 70°C und eine Fußtemperatur T_{F} ≤ 150°C, insbesondere ≤ 120°C, beträgt, wobei insbesondere die Maximaltemperatur in der Reaktionszone T_{MAX} ≥ 1200°C beträgt.

4. Messanordnung nach einem der vorangehenden Ansprüche, wobei das Reaktionsmodul zum überwiegenden Teil mit einer vertikal geschichteten Füllung aus porösen Keramikkugeln befüllt ist, wobei der mittlere Kugeldurchmesser in mehreren Schichten unterschiedlich ist.

5. Messanordnung nach einem der vorangehenden Ansprüche, wobei das Reaktionsmodul eine sich mindestens über die Länge der Reaktionszone erstreckende Innenwandung aus Aluminiumoxid oder Edelstahl und/oder im Kopf- und Fußbereich jeweils einen verstärkten FCKW-Einsatz mit einem eine Teflon-Ummantelung aufweisenden O-Ring als Dichtelement aufweist.

6. Messverfahren zur Bestimmung eines Inhaltsstoffes und/oder eines Qualitätsparameters von Wasser oder Abwasser durch thermischen Aufschluss einer Probe des Wassers oder Abwassers mit definierter Menge in einem Reaktionsmodul, Zuführung des Reaktionsproduktes zu einem Detektor in einem Trägergasstrom und Auswertung eines Detektorsignals zur Ableitung eines Wertes des Inhaltsstoffes oder Qualitätsparameters,
wobei das Reaktionsmodul ein langgestrecktes, im Betrieb vertikal ausgerichtetes Gefäß mit Widerstands- oder Infrarotheizung ist und einen Kopfabschnitt, in dem die Probe eingetragen wird, eine Reaktionszone, in der der thermische Aufschluss erfolgt, sowie einen Fußabschnitt hat, aus dem das Reaktionsprodukt im Trägergasstrom ausgetragen wird, wobei das Reaktionsmodul in einer Zwei-Zonen-Konfiguration eine erste, obere Heizzone (11a), die zur Erreichung eine Maximaltemperatur von 800°C konfiguriert ist, und eine zweite, untere Heizzone (11b) aufweist, die zur Erreichung einer Maximaltemperatur von 1250°C konfiguriert ist,
**dadurch gekennzeichnet, dass**
die erste und zweite Heizzone über hohlzylindrisch um den entsprechenden Abschnitt des Reaktionsmoduls angeordnete Heizmodule (11c, 11d) aus einer hochtemperaturfesten Speziallegierung beheizt sind,
und dass
die Heizmodule (11c, 11d) unterschiedlich dicke mikroporöse Keramikfaser-Isolationen (11e, 11f) aufweisen und auch der Fußabschnitt (11g), ein Bereich (11h) zwischen den Heizzonen und der Kopfabschnitt, (11i, 11j) unterhalb eines Aluminiumdeckels (11k) keramikfaserisoliert sind und das gesamte Reaktionsmodul (11) zusätzlich mit einer makroporösen Außenisolierung (11l) ummantelt ist,
und dass die Widerstands- oder Infrarotheizung und/oder Mittel zur Proben- und Trägergaszuführung derart betrieben werden, dass im Betrieb der Messanordnung eine Kopftemperatur T_{H} ≤ 80°C und eine Fußtemperatur T_{F} ≤ 150°C bei einer Maximaltemperatur in der Reaktionszone T_{MAX} ≥ 1150°C beträgt.

7. Messverfahren nach Anspruch 6, ausgestaltet zur Bestimmung von Stickstoff und/oder Phosphor und/oder des Gehaltes an organischem Kohlenstoff, TOC, oder des chemischen Sauerstoffbedarfs, CSB.

8. Messverfahren nach Anspruch 6 oder 7, wobei die Widerstands- oder Infrarotheizung und/oder Mittel zur Proben- und Trägergaszuführung derart betrieben werden, dass im Betrieb der Messanordnung eine Kopftemperatur T_{H} ≤ 70°C und eine Fußtemperatur T_{F} ≤ 150°C, insbesondere ≤ 120°C, beträgt, wobei insbesondere die Maximaltemperatur in der Reaktionszone T_{MAX} ≥ 1200°C beträgt.

9. Messverfahren nach einem der Ansprüche 6 bis 8, wobei beim Eintragvorgang einer Probe in das Reaktionsmodul ein Einspritz-Eingangssignal für eine Heizsteuereinrichtung der Widerstands- oder Infrarotheizung erzeugt und die Heizsteuereinrichtung derart betrieben wird, dass sie aufgrund des Einspritz-Eingangssignals die Heizleistung der Widerstands- oder Infrarotheizung variiert.

10. Messverfahren nach einem der Ansprüche 6 bis 9, wobei Heizelementen der Widerstands- oder Infrarotheizung mindestens ein Temperatursensor zugeordnet ist und die Signale des oder jedes Temperatursensors entsprechenden Steuereingängen der Heizsteuereinrichtung zugeführt werden und die Heizsteuereinrichtung derart betrieben wird, dass die Heizelemente in Abhängigkeit von einem Ausgangssignal des zugehörigen Temperatursensors und gemäß einem vorbestimmten Temperaturprofil des Reaktionsmodules mit Heizstrom beaufschlagt werden.

11. Messverfahren nach einem der Ansprüche 6 bis 10, wobei in der Heizsteuereinrichtung ein PID-Regelalgorithmus derart betrieben wird, dass dieser zumindest das Ausgangssignal eines Temperatursensors und optional das Einspritz-Eingangssignal zur Temperaturregelung im Reaktionsmodul nutzt.

12. Messverfahren nach einem der Ansprüche 6 bis 11, wobei die Mittel zur Proben- und Trägergaszuführung eine Zuführ-Steuereinrichtung zur automatisch gesteuerten Proben- und Trägergaszuführung aufweisen und derart betrieben werden, dass Schwankungen der Temperatur in der Reaktionszone minimiert werden.

## Claims

1. A measurement arrangement for determining a constituent substance and/or a quality parameter of water or waste water, comprising a reaction module (11) for thermally decomposing a sample of the water or waste water, means for delivering the reaction product to a detector in a carrier gas flow, the detector (42, 45, 47) and an evaluation unit (43, 46, 48) for evaluating a detector signal for deriving a value of the constituent substance or quality parameter,
wherein the reaction module is an elongated vessel of vertical orientation during operation having a resistance heating or infrared heating, and has a head section into which the sample is introduced, a reaction zone, where the thermal decomposition is performed, as well as a foot section, from which the reaction product is output in the carrier gas flow,
wherein the reaction module, the resistance heating or infrared heating, and means for the supply of samples and carrier gas are configured such that during the operation of the measurement arrangement an outer head temperature is T_{H} ≤ 80°C, and an outer foot temperature is T_{F} ≤ 150°C at a maximum temperature in the reaction zone of T_{MAX} ≥ 1150°C, wherein
the reaction module includes in a two-zone configuration a first, upper heating zone (11a) configured to reach a maximum temperature of 800°C, and a second, lower heating zone (11b) configured to reach a maximum temperature of 1250°C,
**characterized in that**
the first and the second heating zones are heated via heating modules (11c, 11d) of a high-temperature resistant special alloy and being arranged in a hollow-cylindrical manner around the corresponding portion of the reaction module, and that the heating modules (11c, 11d) include microporous ceramic fiber insulations (11e, 11f) of different thickness, and also the foot portion (11g), an area (11h) between the heating zones and the head portion (11i, 11j) below an aluminum cover (11k) are ceramic fiber-insulated, and the entire reaction module (11) additionally is coated with a macroporous outer insulation (111).

2. The measurement arrangement according to claim 1, configured for determining nitrogen and/or phosphorus and/or the content of organic carbon, TOC, or the chemical oxygen demand, CSB.

3. The measurement arrangement according to claim 1 or 2, wherein the reaction module, the resistance heating or infrared heating, and means for the supply of samples and carrier gas are configured such that during the operation of the measurement arrangement a head temperature is T_{H} ≤ 70°C, and a foot temperature is T_{F} ≤ 150°C, in particular ≤ 120°C, wherein the maximum temperature in the reaction zone is T_{MAX} ≥ 1200°C in particular.

4. The measurement arrangement according to any one of the preceding claims, wherein the reaction module is mainly filled with a vertically layered filling of porous ceramic balls, wherein the mean ball diameter in several layers is different.

5. The measurement arrangement according to any one of the preceding claims, wherein the reaction module has an inner wall of aluminum oxide or stainless steel extending at least along the length of the reaction zone, and/or in the head and foot areas in each case a reinforced CFC insert having an O-ring with a Teflon sheathing as a sealing element.

6. A measurement method for determining a constituent substance and/or quality parameter of water or waste water by thermal decomposition of a sample of the water or waste water with a defined quantity in a reaction module, delivery of the reaction product to a detector in a carrier gas flow, and evaluation of a detector signal for deriving a value of the constituent substance or quality parameter,
wherein the reaction module is an elongated vessel of vertical orientation during operation having a resistance heating or infrared heating, and has a head section into which the sample is introduced, a reaction zone, where the thermal decomposition is performed, as well as a foot section, from which the reaction product is output in the carrier gas flow, wherein the reaction module includes in a two-zone configuration a first, upper heating zone (11a) configured to reach a maximum temperature of 800°C, and a second, lower heating zone (11b) configured to reach a maximum temperature of 1250°C,
**characterized in that**
the first and the second heating zones are heated via heating modules (11c, 11d) of a high-temperature resistant special alloy and being arranged in a hollow-cylindrical manner around the corresponding portion of the reaction module,
and that
the heating modules (11c, 11d) include microporous ceramic fiber insulations (11e, 11f) of different thickness, and also the foot portion (11g), an area (11h) between the heating zones and the head portion (11i, 11j) below an aluminum cover (11k) are ceramic fiber-insulated, and the entire reaction module (11) additionally is coated with a macroporous outer insulation (111),
and that the resistance heating or infrared heating and/or means for the supply of samples and carrier gas are operated such that during the operation of the measurement arrangement a head temperature is T_{H} ≤ 80°C, and a foot temperature is T_{F} ≤ 150°C at a maximum temperature in the reaction zone of T_{MAX} ≥ 1150°C.

7. The measurement method according to claim 6, designed for determining nitrogen and/or phosphorus and/or the content of organic carbon, TOC, or the chemical oxygen demand, CSB.

8. The measurement method according to claim 6 or 7, wherein the resistance heating or infrared heating and/or means for the supply of samples and carrier gas are operated such that during the operation of the measurement arrangement a head temperature is T_{H} ≤ 70°C, and a foot temperature is T_{F} ≤ 150°C, in particular ≤ 120°C, wherein the maximum temperature in the reaction zone is T_{MAX} ≥ 1200°C in particular.

9. The measurement method according to any one of claims 6 to 8, wherein during the process of introducing a sample into the reaction module, an injection input signal is generated for a heating control device of the resistance heating or infrared heating, and the heating control device is operated such as to vary the heating power of the resistance heating or infrared heating based on the injection input signal.

10. The measurement method according to any one of claims 6 to 9, wherein at least one temperature sensor is assigned to heating elements of the resistance heating or infrared heating, and the signals of the or each temperature sensor are supplied to corresponding control inputs of the heating control device, and the heating control device is operated such as to apply a heating current to the heating elements as a function of an output signal of the associated temperature sensor and in accordance with a predetermined temperature profile of the reaction module.

11. The measurement method according to any one of claims 6 to 10, wherein a PID control algorithm is operated in the heating control device such as to utilize at least the output signal of a temperature sensor, and, optionally, the injection input signal for the temperature regulation in the reaction module.

12. The measurement method according to any one of claims 6 to 11, wherein the means for sample supply and carrier gas supply have a supply control device for automatically controlled sample supply and carrier gas supply and are operated such that temperature fluctuations within the reaction zone are minimized.

## Revendications

1. Agencement de mesure destiné à déterminer un ingrédient et/ou un paramètre de qualité d'eau ou d'eaux usées, comprenant un module de réaction (11) destiné à désagréger thermiquement un échantillon de l'eau ou des eaux usées, des moyens destinés à amener le produit de réaction à un détecteur dans un flux de gaz porteur, le détecteur (42, 45, 47) et un dispositif d'évaluation (43, 46, 48) destiné à évaluer un signal de détecteur pour dériver une valeur de l'ingrédient ou du paramètre de qualité,
sachant que le module de réaction est un récipient à extension longitudinale et à orientation verticale en fonctionnement pourvu d'un chauffage par résistance ou infrarouge et comporte une section de tête dans laquelle l'échantillon est introduit, une zone de réaction dans laquelle la désagrégation thermique a lieu, ainsi qu'une section de pied de laquelle le produit de réaction est extrait dans le flux de gaz porteur,
sachant que le module de réaction, le chauffage par résistance ou infrarouge et les moyens destinés à amener l'échantillon et le gaz porteur sont constitués de telle manière que, en fonctionnement de l'agencement de mesure, une température extérieure de tête TH soit ≤ 80 °C et une température extérieure de pied T_{F} soit ≤ 150 °C à une température maximale dans la zone de réaction T_{MAX} ≥ 1150 °C, sachant que
le module de réaction présente, dans une configuration à deux zones, une première zone de chauffe supérieure (11a) qui est configurée pour atteindre une température maximale de 800 °C et une deuxième zone de chauffe inférieure (11b) qui est configurée pour atteindre une température maximale de 1250 °C,
**caractérisé en ce que**
la première et la deuxième zone de chauffe sont chauffées via des modules de chauffe (11c, 11d) composés d'un alliage spécial résistant aux températures élevées, disposés sous forme de cylindre creux autour de la section correspondante du module de réaction, et **en ce que**
les modules de chauffe (11c, 11d) présentent des isolations en fibres en céramique (11e, 11f) microporeuses d'épaisseur différente et la section de pied (11g), un secteur (11h) entre les zones de chauffe, et la section de tête (11i, 11j), sont également isolés par fibres en céramique en dessous d'un couvercle en aluminium (11k) et l'ensemble du module de réaction (11) est en outre enveloppé par une isolation extérieure (11l) macroporeuse.

2. Agencement de mesure selon la revendication 1, constitué pour déterminer de l'azote et/ou du phosphore et/ou la teneur en carbone organique (TOC) ou la demande chimique en oxygène (CSB).

3. Agencement de mesure selon la revendication 1 ou 2, sachant que le module de réaction, le chauffage par résistance ou infrarouge et les moyens destinés à amener l'échantillon et le gaz porteur sont constitués de telle manière que, en fonctionnement de l'agencement de mesure, une température de tête T_{H} soit ≤ 70 °C et une température de pied T_{F} soit ≤ 150 °C, en particulier ≤ 120 °C, sachant qu'en particulier la température maximale dans la zone de réaction T_{MAX} est ≥ 1200 °C.

4. Agencement de mesure selon l'une des revendications précédentes, sachant que le module de réaction est rempli en majeure partie d'une charge stratifiée verticalement, composée de billes poreuses en céramique, sachant que le diamètre de bille moyen dans plusieurs couches est différent.

5. Agencement de mesure selon l'une des revendications précédentes, sachant que le module de réaction présente une paroi intérieure en oxyde d'aluminium ou en acier inoxydable s'étendant au moins sur la longueur de la zone de réaction et/ou présente dans la section de tête et de pied respectivement un insert en CFC renforcé avec un joint torique présentant une enveloppe en téflon comme élément d'étanchéité.

6. Procédé de mesure destiné à déterminer un ingrédient et/ou un paramètre de qualité d'eau ou d'eaux usées par désagrégation thermique d'un échantillon de l'eau ou des eaux usées à quantité définie dans un module de réaction, amener le produit de réaction à un détecteur dans un flux de gaz porteur et évaluer un signal de détecteur pour dériver une valeur de l'ingrédient ou du paramètre de qualité,
sachant que le module de réaction est un récipient à extension longitudinale et à orientation verticale en fonctionnement pourvu d'un chauffage par résistance ou infrarouge et comporte une section de tête dans laquelle l'échantillon est introduit, une zone de réaction dans laquelle la désagrégation thermique a lieu, ainsi qu'une section de pied de laquelle le produit de réaction est extrait dans le flux de gaz porteur, sachant que le module de réaction présente, dans une configuration à deux zones, une première zone de chauffe supérieure (11a) qui est configurée pour atteindre une température maximale de 800 °C et une deuxième zone de chauffe inférieure (11b) qui est configurée pour atteindre une température maximale de 1250 °C,
**caractérisé en ce que**
la première et la deuxième zone de chauffe sont chauffées via des modules de chauffe (11c, 11d) composés d'un alliage spécial résistant aux températures élevées, disposés sous forme de cylindre creux autour de la section correspondante du module de réaction, et **en ce que**
les modules de chauffe (11c, 11d) présentent des isolations en fibres en céramique (11e, 11f) microporeuses d'épaisseur différente et la section de pied (11g), un secteur (11h) entre les zones de chauffe, et la section de tête (11i, 11j) sont également isolés par fibres en céramique en dessous d'un couvercle en aluminium (11k) et l'ensemble du module de réaction (11) est en outre enveloppé par une isolation extérieure (11l) macroporeuse,
et **en ce que** le chauffage par résistance ou infrarouge et/ou les moyens destinés à amener l'échantillon et le gaz porteur sont exploités de telle manière que, en fonctionnement de l'agencement de mesure, une température de tête T_{H} soit ≤ 80 °C et une température de pied T_{F} soit ≤ 150 °C à une température maximale dans la zone de réaction T_{MAX} ≥ 1150 °C.

7. Procédé de mesure selon la revendication 6, configuré pour déterminer de l'azote et/ou du phosphore et/ou la teneur en carbone organique (TOC) ou la demande chimique en oxygène (CSB).

8. Procédé de mesure selon la revendication 6 ou 7, sachant que le chauffage par résistance ou infrarouge et/ou les moyens d'amener l'échantillon et le gaz porteur sont exploités de telle manière que, en fonctionnement de l'agencement de mesure, une température de tête T_{H} soit ≤ 70 °C et une température de pied T_{F} soit ≤ 150 °C, en particulier ≤ 120 °C, sachant qu'en particulier la température maximale dans la zone de réaction T_{MAX} est ≥ 1200 °C.

9. Procédé de mesure selon l'une des revendications 6 à 8, sachant que lors de l'opération d'introduction d'un échantillon dans le module de réaction, un signal d'entrée d'injection est généré pour un dispositif de commande de chauffe du chauffage par résistance ou infrarouge et le dispositif de commande de chauffe est exploité de manière à faire varier la puissance de chauffe du chauffage par résistance ou infrarouge sur la base du signal d'entrée d'injection.

10. Procédé de mesure selon l'une des revendications 6 à 9, sachant qu'au moins un capteur de température est associé à des éléments de chauffe du chauffage par résistance ou infrarouge et les signaux du ou de chaque capteur de température sont amenés à des entrées de commande correspondantes du dispositif de commande de chauffe et le dispositif de commande de chauffe est exploité de telle manière que les éléments de chauffe soient alimentés en courant de chauffe en fonction d'un signal de sortie du capteur de température associé et selon un profil de température prédéterminé du module de réaction.

11. Procédé de mesure selon l'une des revendications 6 à 10, sachant que dans le dispositif de commande de chauffe, un algorithme de régulation PID est exploité de telle manière que celui-ci utilise au moins le signal de sortie d'un capteur de température et facultativement le signal d'entrée d'injection pour la régulation de température dans le module de réaction.

12. Procédé de mesure selon l'une des revendications 6 à 11, sachant que les moyens destinés à amener l'échantillon et le gaz porteur présentent un dispositif de commande d'amenée pour l'amenée d'échantillon et de gaz porteur à commande automatique et sont exploités de manière à minimiser des variations de la température dans la zone de réaction.
